# EUROPEAN PATENT APPLICATION

(11) **EP 1 839 560 A1**
(43) Date of publication of application: **03.10.2007**
(21) Application number: 06712076.6
(22) Date of filing: 20.01.2006
(51) Int. Cl.: A61B 1/04, H04N 1/00, H04B 5/02, H04J 13/00, H04B 7/26

(54) **MEDICAL APPLICATION COMMUNICATION SYSTEM AND COMMUNICATION METHOD THEREOF**

(30) Priority: 21.01.2005 JP 2005014491
(71) Applicant: Olympus Corporation, Tokyo 151-0072 (JP)
(72) Inventor: IJICHI, Toshiro c/o Int. Property Supp. Dept., 2-3 Kuboyama-cho, Hachioji-shi Tokyo 192-8512 (JP); TAKAHASHI, Kazumasa c/o Int. Property Supp. Dept., 2-3 Kuboyama-cho, Hachioji-shi Tokyo 192-8512 (JP); SUZUKI, Takashi c/o Int. Property Supp. Dept., 2-3 Kuboyama-cho, Hachioji-shi Tokyo 192-8512 (JP); ONO, Yasuhiro c/o Int. Property Supp. Dept., 2-3 Kuboyama-cho, Hachioji-shi Tokyo 192-8512 (JP)
(74) Representative: von Hellfeld, Axel
(86) International application number: PCT/JP2006/300851
(87) International publication number: WO 2006/077966

(57) **Abstract**

A medical communication system is provided with a scope 10 including an image pickup element, and a processor 13 including an image processing section which performs image processing on an image signal obtained by the image pickup element. The processor 13 further includes a communication control section which controls communication with the medical instrument. The scope 10 and the processor 13 are connectable by radio.

## Description

### Technical Field

The present invention relates to a medical communication system and its communication method.

### Background Art

In currently available electronic endoscope systems, a scope section as an imaging unit is connected via a cable to a processor as an image processing unit. Further, the processor is connected via a cable to a peripheral device, such as a monitor/printer as a display/record unit. Jpn. Pat. Appln. KOKAI Publications Nos. 2003-190087 and 6-296589 disclose such electronic endoscope systems.

### Disclosure of Invention

When performing various types of medical surgery using an endoscope, troublesome movements of a processor must be done in a hospital. Concerning, for example, troublesome transfer of the cables of a processor monitor and peripheral devices, and troublesome attachment/detachment of the cables, which must be performed for moving the processor, or constraints on the installation of the instruments, there is a demand for improvements from users.

The present invention has been developed in light of the above, and aims to provide a medical communication system and its communication method for connecting, by radio, the scope to the processor, or the processor to a peripheral unit such as a monitor/printer, thereby eliminating constraints on the installation of the instruments and reducing the time and effort for attaching/detaching cables.

According to a first aspect of the present invention, there is provided a medical communication system comprising: a medical instrument including an image pickup element; and a processor including an image processing section which performs image processing on an image signal obtained by the image pickup element, the processor further including a communication control section which controls communication with the medical instrument,
wherein the medical instrument and the processor are connectable by radio.

According to a second aspect of the present invention, there is provided a medical communication system comprising: a medical instrument including an image pickup element; a processor including an image processing section which performs image processing on an image signal obtained by the image pickup element; and a peripheral unit which performs preset processing using the image signal processed by the processor, the processor further including a communication control section which at least controls communication with the peripheral unit,
wherein the processor and the peripheral unit are connectable by radio.

According to a third aspect of the present invention, there is provided a medical communication system according to a first or a second aspect, wherein a UWB (ultra wideband) wireless transmission system is used for the connection by radio.

According to a fourth aspect of the present invention, there is provided a communication method for use in a medical communication system including: a medical instrument including an image pickup element; a processor including an image processing section which performs image processing on an image signal obtained by the image pickup element; and a peripheral unit which performs preset processing using the image signal processed by the processor, the processor further including a communication control section which at least controls communication with the peripheral unit, the method comprising:
a step of fetching an image signal based on a synchronization signal;
a step of executing image processing on the fetched image signal;
a step of superposing, upon the processed image signal, a control signal for controlling communication with the peripheral unit; and
a step of transmitting, by radio to the peripheral unit, the image signal with the control signal superposed thereon, as a transmission signal.

According to a fifth aspect of the present invention, there is provided a communication method according to a fourth aspect, wherein a UWB (ultra wideband) wireless transmission system is used for the connection by radio.

### Brief Description of Drawings

FIG. 1 is a view illustrating the configuration of a wireless transmission system according to a first embodiment of the invention;
FIG. 2 is a view illustrating the configuration of a wireless transmission system according to a second embodiment of the invention;
FIG. 3 is a view illustrating a configuration example of the processor 13 illustrated in FIGS. 1 and 2;
FIG. 4 is a view illustrating a configuration example of a wireless unit 106; and
FIG. 5 is a timing chart useful in explaining a procedure of transmitting an image signal to a peripheral device by the processor 13.

### Best Mode for Carrying Out the Invention

Embodiments of the invention will be descried in detail with reference to the accompanying drawings.

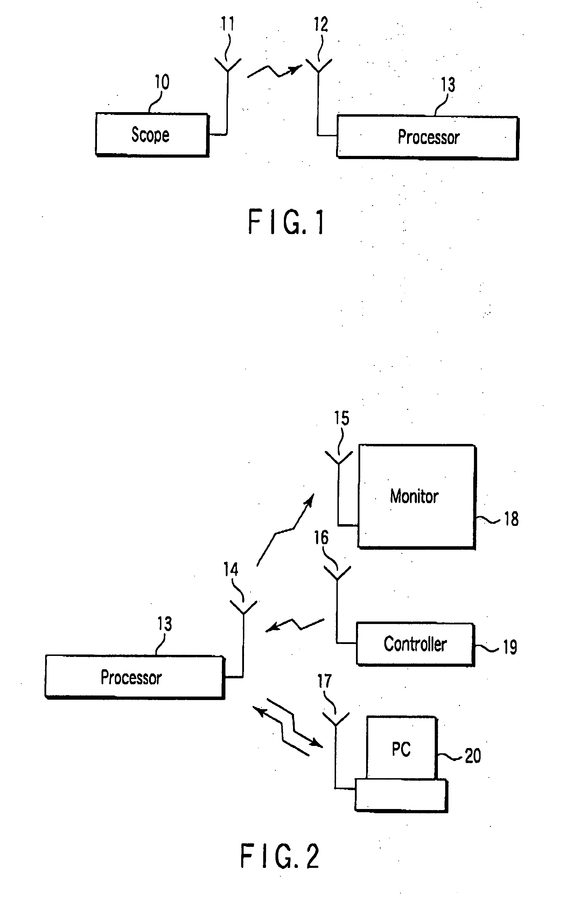

FIG. 1 is a view illustrating the configuration of a wireless transmission system according to a first embodiment of the invention. The first embodiment is characterized in that signals are transmitted by radio via antennas 11 and 12 between a processor 13 for image processing and a scope 10 as a medical instrument, which is inserted for observation into the body cavity of a patient and contains an image pickup element, such as a CCD (charge-coupling device). In this wireless transmission, it is assumed that image data obtained by the scope 10 is transmitted to the processor 13. Further, compact antennas such as dipole antennas are suitable as the antennas 11 and 12.

With the above configuration, the scope 10 can be moved freely, which the constraints on the installation of the instruments can be eliminated, and the time and effort for attaching/detaching, for example, cables can be reduced.

FIG. 2 is a view illustrating the configuration of a wireless transmission system according to a second embodiment of the invention. The second embodiment is characterized in that signals are transmitted by radio via an antenna 14 and antennas 15, 16 and 17 between a processor 13 for image processing and a peripheral device, such as a monitor 18, controller 19 or PC (personal computer) 20. In this wireless transmission, it is assumed that image data processed by the processor 13 is sent to a peripheral device as shown in FIG. 2, where it is subjected to preset processing. Further, compact antennas such as dipole antennas are suitable as the antennas 11 and 12. A printer and VTR can be regarded as other peripheral devices.

With the above configuration, the installation place of the monitor 17 can be set freely, which enables, for example, surgery to be monitored in a room other than the surgery room. Further, the processor 13 can be controlled remotely by the controller 19 if they are connected by radio.

In the embodiment, it is necessary to employ a wireless transmission system that can perform large-capacity interactive communication. For instance, a UWB (ultra wideband) wireless transmission system is suitable.

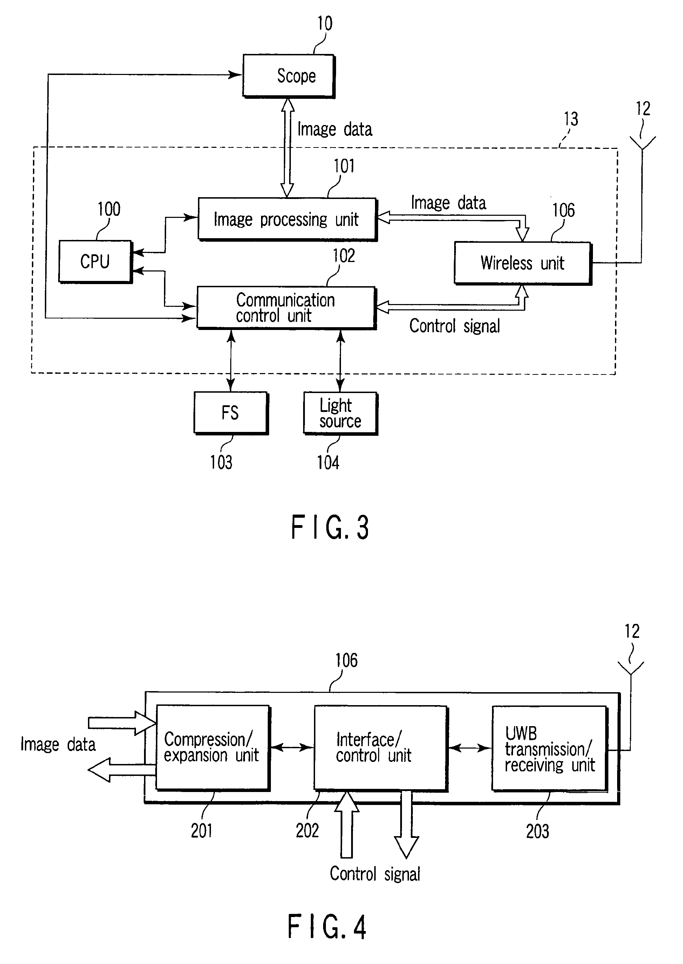

FIG. 3 is a view illustrating a configuration example of the processor 13 described in the first and second embodiments. As shown, the processor comprises an image processing unit 101 for performing preset image processing on image data acquired by the image pickup element in the scope 10; a wireless unit 106 for performing, via an antenna 12 with external peripheral devices, wireless communication that includes image data transmission; a communication control unit 102 for controlling the operation of the wireless unit 106, and a CPU 100 for controlling the operations of the image processing unit 101 and communication control unit 102. The processor 13 is connected to a foot switch (FS) 103, light source 104, etc., as well as to the above-described scope 105. The communication control unit 102 determines the time when the processor can access a peripheral device, and image data is transmitted from the antenna 12 based on the determined time.

FIG. 4 is a view illustrating a configuration example of the wireless unit 106. As shown, the unit comprises a compression/expansion unit 201, interface/control unit 202, and UWB transmission/receiving unit 203. The compression/expansion unit 201 utilizes, for example, JPEG 2000 as a compression/expansion scheme, regarding real-time image processing as important. The interface/control unit 202 performs switching of image data to be sent or a control signal, and controls the UWB transmission/receiving unit 203 and compression/expansion unit 201.

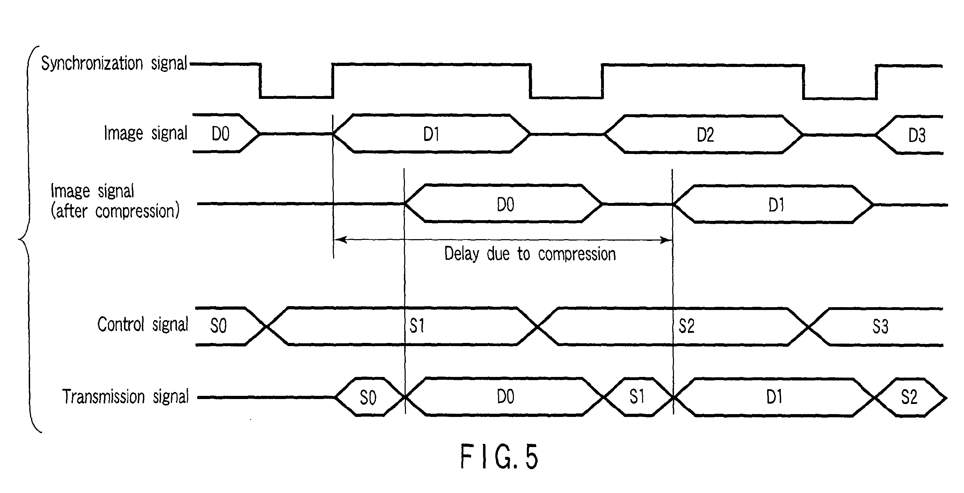

FIG. 5 is a timing chart useful in explaining a procedure of transmitting an image signal to a peripheral device by the processor 13. In the procedure of FIG. 5, an image signal is fetched in accordance with a synchronization signal output at preset intervals during a vertical blanking period for scanning. This image signal is subjected to compression processing. As a result of compression processing, a preset compression delay occurs in the image signal.

On the other hand, the control signal is sequentially output. When transmitting image signals, the control signal is superposed between the image signals, whereby the image signals are transmitted as transmission signals.

### Industrial Applicability

In the present invention, since the scope section and processor, or the processor and a peripheral unit, such as a monitor/printer, are connected by radio, there are no constraints on instrument installation, and the time and effort for attaching/detaching cables can be reduced.

## Claims

1. A medical communication system comprising: a medical instrument including an image pickup element; and a processor including an image processing section which performs image processing on an image signal obtained by the image pickup element, the processor further including a communication control section which controls communication with the medical instrument,
wherein the medical instrument and the processor are connectable by radio.

2. A medical communication system comprising: a medical instrument including an image pickup element; a processor including an image processing section which performs image processing on an image signal obtained by the image pickup element; and a peripheral unit which performs preset processing using the image signal processed by the processor, the processor further including a communication control section which at least controls communication with the peripheral unit,
wherein the processor and the peripheral unit are connectable by radio.

3. The medical communication system according to claim 1 or 2, wherein a UWB (ultra wideband) wireless transmission system is used for the connection by radio.

4. A communication method for use in a medical communication system including: a medical instrument including an image pickup element; a processor including an image processing section which performs image processing on an image signal obtained by the image pickup element; and a peripheral unit which performs preset processing using the image signal processed by the processor, the processor further including a communication control section which at least controls communication with the peripheral unit, the method comprising:
a step of fetching an image signal based on a synchronization signal;
a step of executing image processing on the fetched image signal;
a step of superposing, upon the processed image signal, a control signal for controlling communication with the peripheral unit; and
a step of transmitting, by radio to the peripheral unit, the image signal with the control signal superposed thereon, as a transmission signal.

5. The communication method according to claim 4, wherein a UWB (ultra wideband) wireless transmission system is used for the connection by radio.
